# EUROPEAN PATENT APPLICATION

(11) **EP 3 231 452 A1**
(43) Date of publication of application: **18.10.2017**
(21) Application number: 17166127.5
(22) Date of filing: 11.04.2017
(51) Int. Cl.: A61L 26/00, A01N 47/44

(54) **WOUND IRRIGATION SOLUTIONS**

(30) Priority: 11.04.2016 US 201662320824 P
(71) Applicant: DiCosmo, Frank, Toronto, Ontario M5E 0A4 (CA)
(72) Inventor: DiCosmo, Frank, Toronto, Ontario M5E 0A4 (CA)
(74) Representative: D Young & Co LLP

(57) **Abstract**

An isotonic irrigation solution for wounds, surgical incisions and as a lavage, the irrigation solution comprising saline, antimicrobial, chelator, surfactant and collagenous biological enhancer.

## Description

### Field of the Invention

The present invention relates to irrigation solutions for tissues. More specifically, the invention relates to irrigation solutions where the irrigation solution is aqueous, antimicrobial and biologically enhanced such that it may have use in a variety of applications for wounds and surgical incisions in mammals.

### Background of the Invention

Wound irrigation with a solution applied over an open wound surface achieves wound hydration, removes debris, cleanses the wound, provides nourishment and assists with visual examination for adequate surgical debridement. The irrigation solution removes cellular debris and necrotic tissue, surface pathogens and helps to remove attached microbial biofilms, or residue or attached remnants of topically applied wound care products. Compared to swabbing or bathing, wound irrigation is considered to be the most consistently effective method of wound cleansing.

During normal wound healing, vascular and cellular responses remove pathogenic organisms, cellular debris, and necrotic tissue from the wound during the inflammatory phase. However, in compromised wound healing, the body's ability to adequately heal is impaired because of wound chronicity, delayed angiogenesis and granulation tissue formation, as well as wound surface contamination and infection. In wounds contaminating microorganisms can upset collagen synthesis and modify matrix metalloproteinase activity resulting in poor or impaired healing and, sometimes chronic wounds or stalled wound healing. Irrigation combined with cleansing and debridement of the wound bed facilitates progression from the inflammatory to proliferative phase of wound healing by removing debris and microbes that can impede the healing process. The goal of irrigation is to clean the wound of debris, plantonic and biofilm microbes thus reducing trauma to the wound bed and preparing the wound for adequate debridement.

U.S. 4,504,493 discloses surgical solutions composed of glycerin and water. Other irrigation solutions are also known that contain a variety of salts, including sodium, calcium, potassium or magnesium. Thus electrolyte solutions are known, such as Ringer's solution, a combination of sodium, calcium and potassium ions with sodium lactate, and a variety of balanced salt solutions, many of which employ buffering systems. Also known are other solutions, such as those containing glutathione, sodium bicarbonate, dextrose and the like. U.S. 5,304,724 discloses a formulation for and method of making certain such solutions.

WO1994027440 discloses use of aqueous polyhexamethylene biguanide (PHMB) in lactate-free Ringer solution or saline as a wound antiseptic and/or as a wound treatment agent. CA 1,226,213 describes antimicrobial solutions containing PHMB, chlorohexidine and an amine oxide, and also hydroxypropylmethyl cellulose. CA 2,039,457 discloses a solution having PHMB and polyethylene glycol in lactate-free ringers solution useful as an antiseptic for local administration in treating wounds. U.S. 4,786,436 describes wetting solutions for contact lenses comprising PHMB and to which may be added various water soluble cellulose derivatives and collagen solely to lower viscosity of the contact lens wetting solution.

U.S. 4,938,970 describes an irrigation solution for delivery of a local anesthetic such as lidocaine, medivacaine, bupivacaine, and the like. U.S. 5,820,583 discloses a solution for local reduction of pain, inflammation, spasm and restenosis. U.S. 7,066,932 discloses an irrigating solution and method for determining the temperature of tissue structures under application of energy in an electrosurgery procedure.

Choosing an appropriate solution is important in wound irrigation. Current literature generally favors use of normal saline. Many antiseptics and antibiotics have been employed, but the ideal additive is the subject of debate. Antiseptics and antibiotics while employed for irrigation solutions is subject of debate due to cytotoxicity issues. For example, Prontosan^{R}, Lavasept^{R}, and Prontomed^{R} containPHMB ; Microcyn™ uses hypochlorous acid, Anacept^{R} uses sodium hypochlorite and Octenilin^{R} contains octenidine. DE10012026 discloses an aqueous gel containing PHMB, glycerol and hydroxyethyl cellulose, useful as washing or shower gel having decontaminant action or for covering wounds. Antiseptic solutions containing povidone-iodine, chlorhexidine, and hydrogen peroxide may also be used to irrigate wounds, however, due to cytoxicity to tissues these may negatively influence wound healing by negatively affecting cellular activity and impairing granulation tissue formation.

While irrigation solutions as described above may have various therapeutic uses such as reducing microbial contamination, reducing pain, inhibiting inflammatory processes and responses, and for negative pressure systems for enhanced surgical intervention and wound healing, there still remains a need for a wound irrigation solution to try to effect more than a single therapeutic purpose. To this end, there is a need to provide an irrigation solution that has antimicrobial properties whilst preparing the wound bed for active wound healing. By active wound healing is meant enhanced biological activity of host cells directed to wound healing, collagen biosynthesis and reduced enzyme activity of platelets, fibroblast, keratinocytes, smooth muscle endothelial cells while reducing levels of plantonic microbes and microorganisms in biofilm consortia within the wound bed.

Currently, there is no antimicrobial wound irrigation solution that is biologically enhanced that may overcome at least one of the foregoing problems, and may provide additional advantages over solutions presently known.

### Summary of the Invention

The present invention is generally directed to irrigation solutions for tissues that are antimicrobial, aqueous and biologically enhanced. The irrigation solutions of the present invention are optically clear, essentially non-electrolytic, essentially non-antigenic, non-toxic, essentially non-hemolytic and isotonic such that the solutions do not alter body chemistry or affect the viability of body cells when in contact in a wound bed or operative area.

The irrigation solutions of the present invention are inexpensive to manufacture and not viscous or sticky, so that they may be used in substantial amounts. The irrigation solutions are readily and easily sterilizable and may be stored in concentrated form, or as working solutions for extended periods. The irrigation solutions of the invention can be made in advance in which all of the components may be combined well in advance of use so that the final product need not be prepared at the place or time of use.

The irrigation solutions of the invention are useful for wound irrigation and for irrigation of surgical incisions. Wound irrigation is often overlooked in wound care. When a wound is not well irrigated, washed of contaminants or not receiving the amount of fluids it needs, the basic recovery processes needed to help the wound heal: inflammation, cell migration and granulation tissue formation are impeded increasing the chances of infection. Irrigation also helps with debridement. Thus the irrigation solutions of the invention have use in wound and surgical incision care. The irrigation solutions can also be used as a lavage, that is, for irrigation or washing out of an organ or cavity, such as the stomach or intestine.

According to an aspect of the invention is an isotonic irrigation solution comprising:
- saline;
- antimicrobial;
- chelator;
- surfactant; and
- collagenous biological enhancer.

In aspects the saline comprises physiological saline, saline containing magnesium and/or calcium and phosphate buffered saline (PBS).

In aspects the antimicrobial is selected from the group consisting of polyhexamethylene biguanide (PHMB), triclosan, octenidine, PVP-iodine, chlorhexidine digluconate, silver, hypochlorus acid, sodium hypochlorite and any combination thereof.

In aspects the antimicrobial is present in an amount selected from the group consisting of about 0.001 %-0.5 % w/v, about 0.1 %-0.5 % w/v and about 0.1 % w/v.

In aspects the antimicrobial is 0.1% w/v PHMB.

In aspects the chelator has preservative qualities.

In aspects the chelator is selected from the group consisting of ethylenediaine tetra-acetic acid (EDTA), disodium EDTA, trisodium EDTA, ethyleneglycotetraacetic acid, Diethylenetriaminepentaacetic acid (DTPA), N,N-bis(carboxymethyl)glycine (NTA), and combinations thereof.

In aspects the chelator is present in an amount selected from about 0.01mM up to about 50mM; about 1.0 mM to 10mM; and about 1.0mM.

In aspects the chelator is EDTA present in an amount of about 1.0mM.

In aspects the surfactant is selected from the group consisting of betaine, glycerine and combinations thereof.

In aspects the surfactant is present in an amount of up to about 4% by weight.

In aspects the surfactant is present in an amount selected from about 0.1% to about 4%; about 0.1% to about 3%; about 0.1% to about 2.5%; about 1% to about 3% or 2.5%.

In aspects the surfactant is about 2.5% glycerine.

In aspects the collagenous biological enhancer is selected from the group consisting of hydrolyzed collagen, collagen peptides, denatured collagen, monomeric collagen and combinations thereof.

In aspects the collagenous biological enhancer is present in an amount of about 0.001 to about 1.0%, about 0.05% to about 1.0%, about 0.1% and about 0.05%.

In aspects the collagenous biological enhancer is about 0.02% gelatin.

In aspects the collagenous biological enhancer provides exposed RGD motifs for cellular interaction.

In aspects the irrigation solution of the invention further comprises about 0.01% to about 4% glycosaminoglycans.

In aspects the glycosaminoglycans is selected from the group consisting of chondroitin-6-sulphate, hyaluronic acid and mixtures thereof.

In aspects the glycosaminoglycans provided is about 0.05% chondroitin-6-sulphate and about 0.01% hyaluronic acid.

In aspects the irrigation solution of the invention further comprises a thickening agent.

In aspects the thickening agent is hydroxyethylcellulose (HEC).

In aspects the thickening agent is provided in an amount of about 0.01% to about 5%, about 0.1% to about 0.5%, or about 1.0% w/v.

In aspects the irrigation solution of the invention has a pH of about 5.0 to about 7.5, about 6.5 to about 7.5 or about 6.8 to about 7.4.

In aspects the irrigation solution of the invention is sterilized.

In aspects the irrigation solution of the invention is one or more of optically clear, essentially non-electrolytic, essentially non-antigenic, non-toxic, and essentially non-hemolytic.

In aspects the irrigation solution of the invention is for the irrigation of any type of wound, surgical incision or as a lavage.

In aspects the irrigation solution is provided continuously or intermittently to said wound or surgical incision.

According to an aspect of the invention is an antibacterial isotonic irrigation solution comprising:
saline selected from physiological saline, saline containing magnesium and/or calcium and phosphate buffered saline (PBS);
about 0.001 %-0.5 % w/v antimicrobial selected from the group consisting of polyhexamethylene biguanide (PHMB), triclosan, octenidine, PVP-iodine, chlorhexidine digluconate, silver, hypochlorus acid, sodium hypochlorite and any combination thereof;
about 0.01mM up to about 50mM chelator selected from the group consisting of ethylenediaine tetra-acetic acid (EDTA), disodium EDTA, trisodium EDTA, ethyleneglycotetraacetic acid, Diethylenetriaminepentaacetic acid (DTPA), N,N-bis(carboxymethyl)glycine (NTA), and combinations thereof;
about 4% by weight surfactant selected from betaine, glycerine and combinations thereof;
about 0.001 to about 1.0% collagenous biological enhancer selected from the group consisting of hydrolyzed collagen, collagen peptides, denatured collagen, monomeric collagen and combinations thereof;
optionally 0.01% to about 4% glycosaminoglycans; and
optionally about 0.01% to about 5% hydroxyethylcellulose.

According to another aspect of the invention is a method for the irrigation of a wound or surgical incision, by applying the irrigation solution of the invention to wounds or surgical incisions.

According to a further aspect of the invention is a method to maintain the viability of cells/tissues of a wound, to remove non-viable or necrotic tissue of a wound, to remove contamination materials or microbes form a wound, to enhance the removal of biofilms of a wound, to enhance visualization of a wound, to provide tissue nutrients and enzyme inhibitors to a wound, to chelate metal ions of a wound or to establish a wound bed and activate host cells to support healing of said wound, the method comprising; applying the irrigation solution of any of the embodiments described herein to said wound for a time sufficient to achieve any one or more of the foregoing.

According to a further aspect of the invention is a method for making an isotonic irrigation solution comprising admixing: saline; antimicrobial; chelator; surfactant; collagenous biological enhancer; optional glycosaminoglycans and optional thickening agent,wherein said admixed solution is clear and has a pH of about 5.0 to about 7.5.

Aspects of the invention are as follows:
1. An isotonic irrigation solution comprising:
   - saline;
   - antimicrobial;
   - chelator;
   - surfactant; and
   - collagenous biological enhancer providing exposed RGD motifs for cellular interaction.
2. The irrigation solution of claim 1, wherein:
   said saline comprises physiological saline, saline containing magnesium and/or calcium and phosphate buffered saline (PBS);
   said antimicrobial is selected from the group consisting of polyhexamethylene biguanide (PHMB), triclosan, octenidine, PVP-iodine, chlorhexidine digluconate, silver, hypochlorus acid, sodium hypochlorite and any combination thereof, preferably PHMB;
   said chelator has preservative qualities and is selected from the group consisting of ethylenediaine tetra-acetic acid (EDTA), disodium EDTA, trisodium EDTA, ethyleneglycotetraacetic acid, Diethylenetriaminepentaacetic acid (DTPA), N,N-bis(carboxymethyl)glycine (NTA), and combinations thereof, preferably EDTA;
   said surfactant is selected from the group consisting of betaine, glycerine and combinations thereof, preferably glycerine;
   wherein said collagenous biological enhancer is selected from the group consisting of hydrolyzed collagen, collagen peptides, denatured collagen, monomeric collagen and combinations thereof, preferably gelatin.
3. The irrigation solution of claim 1 or 2, wherein said antimicrobial is present in an amount selected from the group consisting of about 0.001%-0.5 % w/v, about 0.1 %-0.5% w/v and about 0.1 % w/v, preferably 0.1 % w/v PHMB.
4. The irrigation solution of claim 1,2 or 3, wherein said chelator is present in an amount selected from about 0.01mM up to about 50mM; about 1.0 mM to 10mM; and about 1.0mM, preferably about 1.0mM EDTA.
5. The irrigation solution of any one of claims 1 to 4, wherein said surfactant is present in an amount selected from up to about 4% by weight, about 0.1 % to about 4%; about 0.1 % to about 3%; about 0.1 % to about 2.5%; about 1% to about 3% or 2.5%, preferably 2.5% glycerine.
6. The irrigation solution of any one of claims 1 to 6, wherein said collagenous biological enhancer is present in an amount of about 0.001 to about 1.0%, about 0.05% to about 1.0%, about 0.1% , about 0.05% or about 0.02%.
7. The irrigation solution of any one of claims 1 to 5, wherein said collagenous biological enhancer facilitates wound/incision healing.
8. The irrigation solution of any one of claims 1 to 7, further comprising about 0.01% to about 4% glycosaminoglycans selected from the group consisting of chondroitin-6-sulphate, hyaluronic acid and mixtures thereof.
9. The irrigation solution of claim 8, wherein said glycosaminoglycans provided is 0.05% chondroitin-6-sulphate and 0.01% hyaluronic acid.
10. The irrigation solution of any one of claims 1 to 9, further comprising a thickening agent, preferably hydroxyethylcellulose (HEC).
11. The irrigation solution of claim 10, wherein said thickening agent is provided in an amount of about 0.01% to about 5%, about 0.1% to about 0.5%, or about 1.0% w/v.
12. The irrigation solution of any one of claims 1 to 11 having a pH of about 5.0 to about 7.5, about 6.5 to about 7.5 or about 6.8 to about 7.4, further wherein said solution is one or more of sterilized, anti-microbial, biologically enhancing, optically clear, essentially non-electrolytic, essentially non-antigenic, non-toxic, and essentially non-hemolytic.
13. The irrigation solution of any one of claims 1 to 12, provided in a ready to use format, or in a concentrated format and/or provided soaked in a suitable material selected from gauze and sponge and optionally packaged for use.
14. The irrigation solution of any one of claims 1 to 13, provided as a kit with instructions for use.
15. A method to maintain the viability of cells/tissues of a wound, to remove non-viable or necrotic tissue of a wound, to remove contamination materials or microbes form a wound, to enhance the removal of biofilms of a wound, to enhance visualization of a wound, to provide tissue nutrients and enzyme inhibitors to a wound, to chelate metal ions of a wound or to establish a wound bed and activate host cells to support healing of said wound, the method comprising; applying the irrigation solution of any one of claims 1 to 14 to said wound for a time sufficient to achieve any one or more of the foregoing.

### Description of the Invention

Tissue irrigation solutions are provided herein that may be employed in any surgical procedure , as a lavage, or for wound bed preparation (wound care) utilizing irrigation as a means of wetting tissue to help with one or more of maintaining viability, removing non-viable or necrotic tissue, removing contaminating materials or microbes and enhancing the removal of biofilms, distension of a working space, enhancing visualization, providing tissue nutrients and enzyme inhibitors, chelating metal ions and also helping to establish a wound bed and active host cells that support wound healing.

More specifically, the irrigation solutions of the invention are aqueous-based isotonic physiological solutions that are antimicrobial, comprise a metal chelator and a collagenous biological enhancer, and may provide the above mentioned features and benefits. The antimicrobial and biologically enhanced irrigation solutions are for use on wound areas and surgical incisions; may be used as a wash or lavage for chronic, traumatic wounds, bums, lacerations and acute wounds and the like that may be contaminated with microbes, or for preparation of the wound bed for further treatment, such as application of a wound dressing, or surgical debridement of the wound or other procedure. The antimicrobial and biologically enhanced irrigation solutions of the invention also have use for sores on the skin or a mucous membrane, accompanied by the disintegration of tissue, the formation of pus, etc.

The solutions of the present invention may be suitably applied to both humans and non-human mammals.

Accordingly, definitions are provided where certain terms related to the invention are defined specifically for clarity, but all of the definitions are consistent with how a skilled artisan would understand these terms. Particular methods and materials are described, although any methods and materials similar or equivalent to those described herein can be used in the practice of the invention. All references referred to herein are incorporated by reference herein in their entirety. It is also to be understood that the terminology used herein is for the purpose of describing particular aspects only, and is not intended to be limiting.

As used herein a "wound" is generally an injury to living tissue usually involving division of tissue or rupture of the integument or mucous membrane, caused by a cut, blow, or other impact, typically one in which the skin is cut or broken. A wound is characterized by being due to external violence or some mechanical agency rather than disease. As used herein, the term "wound", also includes surgical wounds and operative/interventional sites. Wounds also include chronic wounds, diabetic foot ulcers, tissue ulcers, diabetic skin ulcers, pyroderma gangrenosum, epidermolysis bullosa may result from some form of disease or immune dysfunction including but not limited to diabetes and ischemia.

As used herein "surgical incision" is generally a cut made through the skin to facilitate an operation or procedure inclusive of arthroscopic procedures. Often, multiple incisions are possible for an operation. In general, a surgical incision is made as small and unobtrusive as possible to facilitate safe and timely operating conditions.

As used herein, the term "saline" refers to salt, and more particularly, to sodium chloride.

As used herein, the term "isotonic" refers to a solution having the same osmotic pressure as some other solution, especially one in a cell or a body fluid.

As used herein, the term "lavage" refers to irrigation or washing out of an organ or cavity, as of the stomach or intestine.

As used herein, the term "osmotic activity" refers to the net diffusion of water across a selective permeable membrane that is permeable in both directions to water, but varying permeable to solutes, wherein the water diffuses from one solution into another of lower water potential.

By "pharmaceutically acceptable" is meant a material or materials that are suitable and not biologically or otherwise undesirable, i.e., that may be administered to an individual without causing any undesirable biological effects or interacting in a deleterious manner.

By the terms "effective amount or concentration" or "therapeutically effective amount" as provided herein are meant a nontoxic but sufficient amount of the solution to provide the desired therapeutic effect. An appropriate "effective" amount in any individual case may be determined by one of ordinary skill in the art using only routine experimentation.

The irrigation solutions of the invention comprise saline; antimicrobial; chelator; glycerin; hydrolyzed and/or collagen peptide; optionally denatured or monomeric collagen; optionally a thickening agent; and optionally glycosaminoglycans.

The irrigation solutions of the invention are aqueous-based, physiologic solutions comprising normal saline, such as for example a solution of 0.90% w/v of NaCl, 308 mOsm/L or 9.0 g per liter. The solution is referred to as physiological saline or isotonic saline. Sterile isotonic saline is used in one aspect of the invention, in other aspects phosphate buffered saline (PBS) may also be used or a saline that contains calcium or magnesium. One of skill in the art would understand the type of isotonic solution to use in the present invention so long as it is isotonic and sterile.

An antimicrobial is added to the isotonic solution, saline, in an amount of about 0.001-0. 5 %. In aspects the amount may be about 0.1 %-0.5 or may be about 0.1 %. Any ranges therebetween are encompassed within these noted ranges. The antimicrobial for use in the present invention may be selected from the group consisting of polyhexamethylene biguanide (PHMB also known as polihexanide), triclosan, octenidine, PVP-iodine, chlorhexidine digluconate, silver, hypochlorus acid, sodium hypochlorite and any combination thereof. In aspects, PHMB is used in the irrigation solutions of the invention. PHMB is commercially available as COSMOCIL CQ from Lonza. Other sources of PHMB are suitable such as Vantocil IB from Arch Chemicals Inc. USA, and other sources generally known to those in the art.

Furthermore, a chelator is added to the saline. Chelating agents increase antiseptic effects and thereby may render the irrigation solution essentially more effective in preventing infection, and as a preservative in the solution. A chelator is added in the amount of 0.01mM up to about 50mM, and in aspects about 1.0 mM to 10mM, and in aspects 1.0mM. Suitable chelators for use in the present invention are selected from the group consisting of ethylenediaine tetra-acetic acid (EDTA), disodium EDTA, trisodium EDTA, ethyleneglycotetraacetic acid (EGTA also known as egtazic acid), diethylenetriaminepentaacetic acid (DTPA), N,N-bis(carboxymethyl)glycine (NTA) and combinations thereof.

In aspects, EDTA is used in the irrigation solutions of the invention. EDTA and is widely available as recognized by one of skill in the art. EDTA is also a preservative and works well in combination with PHMB. EDTA as a preservative is a known inhibitor of matrix metalloproteases (MMPs) as it chelates zinc ion from the active site of MMPs rendering them inactive. EDTA acts to chelate cations, and thereby inhibits enzymatic activity, known to contribute to wound inflammation. Further, EDTA provides anti-biofilm ability by chelating calcium and magnesium ions, which help maintain the physical integrity of the biofilm. EDTA also removes iron ions vital to microbial virulence and pathogenicity. Its property as a chelating agent is due to its ability to sequester di- and tri-cationic metal ions such as Ca²⁺ and Fe³⁺. After binding to EDTA, metal ions remain in solution, and have diminished activity as they are extensively enveloped and, as a consequence, their catalytic properties are often suppressed. Thus, EDTA abstracts Zn²⁺ ions from the active site of gelatinases, MMP 2 and MMP 9, thereby inactivating the enzymes.

EDTA, such as disodium and trisodium EDTA, and in particular tetra-sodium EDTA, have been shown to have antimicrobial and anti-biofilm abilities. It is a good potentiating and synergistic agent when used in conjunction with PHMB and other antimicrobial agents. Gram-negative bacterial cell walls in particular are disrupted with EDTA. The extracellular polysaccharide matrix of microbial biofilms that makes up approximately 80% of the biofilm structure, is disrupted by EDTA. [Finnegan & Percival EDTA: An Antimicrobial and Antibiofilm Agent for Use in Wound Care. ADVANCES IN WOUND CARE, 4:415-421 (2015)].

To the irrigation solution is also added a surfactant (surface active agent) which reduces the surface tension of a fluid allowing it to better wet a surface. Denatured collagen (gelatin) is generally known to have surface active properties. Any amphoteric surfactant having this property can be utilized in the irrigation solutions of the invention such as betaine and glycerin. The amphoteric surfactant is present in an amount of up to about 4%. In aspects about 0.1% to about 4% w.v. In aspects up to about 3.5%, up to about 3%, up to about 2.5%, up to about 2%, up to about 1.5%, up to about 1%, up to about 0.5%. In aspects the irrigation solutions comprise glycerin, in aspects about 2.5% glycerin is utilized.

Further, to the irrigation solution is added collagenous biological enhancer in an amount of about 0.001 to about 1.0%, in aspects about 0.001 to about 1.0%, about 0.1 % and about 0.05%. Such collagenous biological enhancer may be selected from hydrolyzed collagen, collagen peptides, denatured collagen, monomeric collagen and combinations thereof. The collagenous biological enhancer is such that it provides RGD sequence(s) that are exposed and thus have the ability to interact with cells and/or tissues. The collagenous biological enhancer helps to attach to cell and tissue types to support wound/incision healing. When gelatin is used in aspects it is provided at 0.02%.

In wounds, the extracellular matrix (ECM) is a complex structure in the body that surrounds and supports cells. *In vivo,* cells, including stem cells reside within the ECM, receive and respond to physical and biochemical signals from neighbouring cells, ECM components and tissues. The ECM consists of several structural proteins such as collagen, laminin, fibronectin, vitronectin, and elastin that are susceptible to degradation and reassembly. The most abundant of the ECM proteins is collagen.

Collagen and other structural proteins of the ECM are often associated with proteoglycans and glycoproteins. These molecules provide signaling that regulate cellular response and cell behavior. Cells bind to the ECM via integrins. Integrins link the internal cellular cytoskeleton to the external ECM through cytoplasmic bridging proteins, and mediate the cells' ability to sense the ECM-environment and respond accordingly. Increased or even reduced cell adhesion via integrins has determinative effects on cellular metabolism and function. It is now widely accepted that the ECM and collagen participate in regulation of the function of cells within the ECM. Interactions between cells and the extracellular matrix coordinate signaling pathways that control various aspects of cellular behavior. Integrins sense the physical properties of the extracellular matrix and organize the cytoskeleton accordingly (Huveneers & Danen, Journal of Cell Science, 122: 1059-1069, 2009).

Native collagen has a triple helical structure and forms microfibrils. A microfibril is composed of many tropocollagen helices, and each of these is assembled from three polypeptide chains twisted together to form the triple helical, native structure composed of amino acids. At certain locations within each of the three polypeptide chains, there are specific repeated amino acid sequences of arginine-glycine-aspartate (RGD, where R is arginine, G is glycine, and D is aspartate). Triple helical type I collagen contains two RGD moieties in each of its two α1 chains and 4 RGD units in its α2 chain for total of 8 RGD motifs. The RGD sequences are twisted within the triple helix structure of native collagen and are thus not readily exposed on the surface of triple helical native collagen molecules. The RGD sequences are thus hidden or "cryptic" within the native structure of collagen and not readily contacted by cells exposed to triple helical collagen in its native or non-denatured conformation. Thus, in the native form of collagen, cells such as fibroblasts, endothelial cells, platelets and stem cells, do not readily interact through the cells' αvβ3 integrins with the RGD tripeptide motifs. As well, flow-cytometric phenotyping and immunofluorescence phenotyping show that αv, αvβ3 and αvβ5 are expressed in all mononuclear cells (muscle precursors and interstitial cells) seeded on extracellular molecules such as gelatin, vitronectin and fibronectin (Sinanan et al. 2008, αvβ3 and αvβ5 integrins and their role in muscle precursor cell, adhesion. Biol. Cell. 100, 465-477).

Integrin binding RGD sites have been identified in collagen and many other ECM proteins, including vitronectin, fibrinogen, von Willebrand factor, collagen, laminin, osteopontin, tenascin and bone sialoprotein, as well as in membrane proteins. Certain integrins have been shown to bind to ECM molecules in a RGD dependent manner: α3β1; α5β1; α8β1; αIIβ3; αvβ1;αvβ3; αvβ5; αvβ6; αvβ8; and to some extent α2β1 and α4β1(Hersel et al. Biomaterials 24:4385-4415, 2003). A predominant role of the RGD site is for endothelial cell adhesion and for binding of αvβ3 and αvβ5 integrins (Pedchenko et al. 2004, αvβ3 and αvβ5 integrins bind both the proximal RGD site and non-RGD motifs within noncollagenous (NC1) domain of the α3 chain of type IV collagen: implication for the mechanism of endothelia cell adhesion. J Biol Chem., 23: 279). Since RGD motifs were found to promote cell adhesion and influence cell survival and cell metabolism, numerous polymers have been chemically functionalized with RGD peptides, including collagen for biomedical applications, including but not limited to tissue regeneration (Niu et al. J. Mater. Sci Technol. 21: 571-576,2005; Hersel et al. 2003).

Damage or trauma to collagen, such as thermal injury or proteolytic attack *in vivo* by gelatinase enzymes, leads to the unwinding of native or triple-helical collagen. The scission and subsequent unwinding, also known as "denaturation" of native collagen renders it susceptible to further degradation and fragmentation into various peptides by proteases (eg, elastase, collagenases, gelatinases secreted by neutrophils, macrophages, fibroblasts, and keratinocytes, and stem cells) and more specifically by MMP2 and MMP9 gelatinases. Denatured collagen (also known as gelatin) and degraded collagen molecules leads to exposure of the RGD sequences that become biologically active and influence biochemical and cell interactions at these RGD-binding sites. Native collagen is generally remodelled in the body by collagenases and gelatinases that serve to expose the RGD moieties with resultant biological activity toward competent cells, including platelets, fibroblasts, keratinocytes, and stem cells. Other cells may interact with RGD, through integrin-mediated contact.

Cells bind to native collagen using the α1β1, α10β1, α11β1, and α2 β1 integrin receptors that are not "cryptic" or hidden within the helical structure of native type 1 collagen. On the other hand, partial or total denaturation of collagen type 1, reveals cryptic RGD motifs that are recognized by αvβ3, αvβ1 and α5β1 receptors as well as the αIIB β3 integrins on platelets. Therefore, cells show different biological activities, or may remain quiescent, depending on whether or not, their surface integrins contact and bind with, either native collagen or denatured collagen as each of these collagens will have distinct amounts and differing locations of unexposed and exposed bioactive amino acid sequences. For example, fibroblasts produce collagen for secretion and ECM remodelling, and assist in wound healing, bind the RGD sequence with *α*v*β*3 integrins.

In industry, denatured collagen, is derived from native collagen in the presence of extreme heat, acid and base treatments that result in loss of the triple helical structure of collagen and degradation and hydrolysis of the polypeptide chains of collagen. Hydrolysis of monomeric collagen and denatured collagen can be readily accomplished using enzymatic means generally well-known in the art. Generally, the loss of the triple helical configuration (denaturing) of collagen is considered an adverse reaction to be avoided when purifying native collagen for biomedical applications such as tissue regeneration.

Wound healing requires angiogenesis, and revascularization or new blood vessel formation, and involves the processes of adhesion, invasion, migration, proliferation, and capillary tube formation by specific cells. New blood vessels grow from endothelial cells. Angiogenesis requires specific molecular interactions between vascular cells and components of the ECM. Microvascular endothelial cell surface receptors that are specific for the fibrin-rich provisional ECM are believed to be involved in angiogenesis. There are several integrin receptors, but only αvβ3 can recognize and bind all the provisional matrix proteins, including fibrin and fibronectin.

Because of the bioactivity of the RGD sequence and certain synthesized mimics of RGD, any source of RGD peptides, from hydrolyzed collagen, denatured collagen or collagen peptides with exposed RGD peptides is used as the collagenous biological enhancer in the irrigation solutions of the invention.

The source of the denatured collagen, also known as gelatin, may be bovine, porcine, equine, or fish or other mammalian source. The source of the collagen having RGD peptides is not limiting and any suitable source can provide RGD peptides for this invention.

Gelatin is a biocompatible polymer, and is used as a drug delivery vehicle for release of active biomolecules and in manufacture of scaffolds for tissue engineering applications and in manufacture of collagen wound dressings. Gelatin is a heterogeneous mixture of water-soluble proteins of high average molecular masses derived from collagen. It is extracted by boiling skin, tendons, ligaments, bones, etc. in water. Type A gelatin is derived from acid-treated tissue and Type B gelatin is derived from lime-treated tissue. Gelatin is soluble in glycerol and more soluble in hot than in cold water. Suitable commercial sources are from Gelita corporation, Vyse Gelatin, Rousellot Gelatin, Great Lakes Gelatin, Nitta Gelatin, or other commercial sources. Gelatin can be hydrolyzed to yield collagen fragments generally in the range of 2-5 kDa (kilodaltons), and the size range is suitable for the irrigation solutions of the invention. Unhydrolyzed collagen and native collagens are also suitable to practice the invention. Collagens derived from any source, porcine, bovine, equine, avian, fish, amphibian, and from genetically modified sources, such as yeast and bacterial sources, whether denatured, hydrolyzed or in native forms are suitable. In aspects, a source of the collagens of the current invention are bovine, and porcine Type 1 collagens, and avian Type 2 collagen derived from avian sterum cartilage from BioCell Collagen.

Optionally, the irrigation solutions of the invention may include glycosylaminoglycans, such as chondroitin sulphate and hyaluronic acid in the range of about 0.01 to about 4%. In aspects these are chondroitin-6-sulfate and/or hyaluronic acid. In aspects chondroitin-6-sulfate (0.05% (w/v); Sigma, San Jose, CA, USA) and hyaluronic acid (0.1% (w/v); Sigma) are representative non-limiting examples.

Optionally, a thickening agent (non-ionic flow modifier) is added to the irrigation solutions of the invention in an amount of about 0.01 to about 5.0% w/v. In aspects the amount is about 0.1% to about 0.5%, in further aspects about 1.0% w/v. Suitable thickening agents may include hydroxyethylcellulose which is a non-ionic flow modifier derived from cellulose. Hydroxyethylcellulose when added to an aqueous solution changes the flow properties of water. It is of benefit as it is enhances cleansing and modifies foaming in aqueous irrigant systems. Of all the natural polymers hydroxyethylcellulose available from Dow Chemical Company creates one of the most clear systems and makes an excellent vehicle for active ingredients in solutions when crystal clear solutions are required. It allows the use of less surfactant and also reduces the irritation potential to sensitive tissues making the wound cleansers more gentle and effective. It may be added to the aqueous phase at the end of the formulation process with moderate agitation and it will hydrate very efficiently with very little mixing.

One of ordinary skill in the art will readily be able to select an appropriate type and amount of the selected isotonic solution, antimicrobial, chelator, surfactant, collagenous biological enhancer, optional thickening agent, and optional glycosylaminoglycans for inclusion in wound irrigation solutions of the invention that are pharmaceutically acceptable in any effective amount or concentration or therapeutically effective amount to achieve the desired result to the wound or surgical excision site.

To summarize the present invention is applicable for wash in surgical incision sites, acute wound, traumatic wounds, tissue ulcers, foot ulcers, chronic wounds, bums, lacerations and the like; it provides biologically active compounds (collagenous biological enhancers) like gelatin that contain RGD sequences and EDTA that chelate metallic ions. The presence of an antimicrobial such as PHMB in the lavage helps remove microbial contamination and further provides antimicrobial action in the wound bed and acts as a preservative to the irrigation solutions. In certain wounds like bums, ulcers, traumatic wounds and the like, microbes may be present in large amounts and in consortia known as biofilms. The lavage of the present invention in providing EDTA is known to help eradicate biofilms helping to remove microbial biofilm helps prevent recurring infection of said wounds. In providing RGD sequences the irrigation solution helps increase cellular activity and support wound healing.

The irrigation solutions of the invention can be applied in any known manner as is understood by one of skill in the art. Irrigation includes bulb syringes, piston syringes, pressure canisters, whirlpool agitator, whirlpool hose sprayer, irrigation fluid in plastic containers with a pour cap or nozzle, and pulsed lavage (eg, jet lavage, mechanical lavage, pulsatile lavage, mechanical irrigation, high-pressure irrigation).

Irrigation using the solutions of the invention can be continuous irrigation that is the uninterrupted stream of irrigant to the wound's surface or pulsed irrigation that is the intermittent or interrupted pressurized delivery of an irrigant, typically measured by the number of pulses per second. Power-pulsed lavage is a wound irrigation system that uses an electrically powered pump system to deliver a high volume of irrigation solution under pressure.

The irrigation solutions of the invention can be provided at various pressures as is understood by one of skill in the art. High pressure is often used to describe acute wound irrigation; however, pressure parameters can be varied within this definition. High-pressure lavage, usually performed using syringes and needles, is 35-70 pounds per square inch (psi), and low-pressure irrigation is 1-15 psi, as defined by the American College of Surgeons.

The irrigation solutions of the invention can be provided at any desired volume. Volumes of 50-100 mL per centimeter of laceration length or per square centimeter of a wound have been used. Irrigation volume should be determined according to wound characteristics and degree of contamination as is understood by one of skill in the art. In the case of more contaminated wounds, the wounds should be irrigated until all visible debris is removed. Copious amounts of the solutions of the invention should be used for irrigation and decontamination of chemical bums.

Irrigation, particularly high pressure, can splash and spread bacteria to surrounding areas and people. The use of a plastic shield at the end of the irrigating syringe reduces this hazard. Where needed, a face shield, mask, and protection over scrubs is advised. IV sites and other open areas may be used for protection from splashing as required. In aspects to avoid splash, the irrigation solution of the invention can be provided soaked in suitable material such as gauze, sponge and the like that is used in patient care of wounds and/or incisions and the like. In this embodiment, the suitable material may be soaked and pre-packaged in a water tight packaging that is easy to open and the material soaked in the irrigation solution is ready for use for application. The irrigation solution can drip from the suitable material as placed over a wound for example and/or can be "squeezed" to apply additional soaked irrigation solution. The suitable material is useful for wiping and removing unwanted tissue/cell debris as well.

In any of the embodiments of the invention the irrigation solution can be provided as a kit prepackaged and ready for use either concentration and ready for dilution or simply ready for use in any desired volume and container, squeezable or pre-packaged soaked as detailed above. Instructions for storage and use may be included. Long term storage is refrigerated, but it also keeps for long periods of time at room temperature.

Wounds should be irrigated initially and at each dressing change. All wound surfaces should be irrigated, which may require opening wound edges and flaps for exposure. Wounds may be irrigated again upon re-examination. Pulsed lavage should not be performed over exposed blood vessels, nerves, tendons, or bone.

The irrigation solutions of the invention can be used in combination with hydrodebridement. Hydordebridement is the synchronous application of an irrigant in tandem with debridement. Particularly in the presence of thick exudate, slough, or necrotic tissue, hydrodebridement may aid in cleansing and mechanical debridement. Hydrodebridement may also be used with negative pressure wound therapy (NPWT). Some NPWT systems are equipped with technology that allows automatic instillation of topical solutions to be delivered and removed from the wound site during NPWT. This technology is indicated for patients who would benefit from vacuum-assisted drainage and controlled delivery of topical wound treatment solutions and suspensions over the wound bed. NPWT can be used with the irrigation solutions of the invention for wound cleansing, irrigation, pain, and removal of infectious material.

Antibiotics such as vancomycin, bacitracin, and silver-based solutions (silver nitrate, may be discolouring however) may also be incorporated into the solution as is understood by those of skill in the art.

The irrigation solutions of the invention can be made and stored refrigerated and used at any desired temperature such as at about 37°C and pH is desirably at about 5.0 to about 7.5, in aspects, about 6.5 to about 7.5 and in further aspects about 6.8 to about 7.4.

The solution of this invention may be sterilized by autoclaving for 20 min, 121°C, liquid cycle. Other suitable sterilization methods, like gamma-ray irradiation may be used. The sterilized solution is stored at room temperature and should be kept from freezing.

To summarize, the present invention provides irrigation solutions used as an irrigant or a lavage that is isotonic, nontoxic, clear and inexpensive; having antiseptic activity (antimicrobial activity) to help reduce bioburden, microbial contamination and help remove biofilms attached to the wound bed; it is non-toxic to tissue and cells necessary for would healing such as platelets, fibroblasts, keratinocytes, and smooth muscle endothelial cells, and should encourage granulation tissue formation; it is non-toxic to cells and helps prepare fibroblasts and other cells to produce collagen and extracellular matrix components needed for wound healing, and endothelial cells for new blood vessel formation; it essentially reduces excess matrix metalloprotease activity (MMP2 and MMP9) in wounds to help minimize unwanted protease activity; and it helps stimulate healthy cells and encourage and support wound healing. The present irrigation solution may meet these needs in a single convenient and inexpensive, clear solution.

In one aspect, the solution may be a 0.9% saline solution comprising:
1) 0.1 % PHMB;
2) 1-10 mM EDTA or 1.0mM acid (EDTA);
3) 0-1%, 1-3%, 2-4% glycerin, or 2.5% glycerin;
4) 0.001-0.1% hydrolyzed collagen or denatured collagen, preferably 0.05% or 0.01-0.1%, and preferably 0.05% collagen peptides. When using denatured collagen, gelatin is preferably added as 0.02%;
5) Chondroitin-6-sulfate (0.05% (w/v); and hyaluronic acid (0.1% (w/v); and
6) 0.01-5% hydroxyethylcellulose (HEC), preferably 0.1-0.5, most preferably 0.1%.

In 5) and 6) the chondroitin-6-sulfate and HEC may be optional.

In one aspect the solution may be PBS comprising:
1) 0.1 % PHMB;
2) 0-50 mM EDTA, optimally 1.0mM acid (EDTA);
3) 0-1%, 1-3%, 2-4% glycerin, or 2.5% glycerin;
4) 0.001-0.1% hydrolyzed collagen or denatured collagen, preferably 0.05%, or 0.01-0.1% collagen peptides. When using denatured collagen, gelatin may preferably added as 0.02%;
5) optionally, Chondroitin-6-sulfate (0.05% (w/v); and hyaluronic acid (0.1% (w/v); and
6) optionally, 0.01-5% Hydroxyethylcellulose, 0.1-0.5, or 0.1%.

In one aspect the solution may be saline comprising:
1) 0.01-0.05 % PHMB, preferably 0.1%-0.5 PHMB and more preferably 0.1% PHMB;
2) 0-50 mM sodium ethylenediamine tetra-acetic acid (EDTA), optimally 10mM (EDTA);
3) 0-1%, 1-4%, 2-3.5 % glycerin, or 2.5% glycerin;
4) 0.1-0.1% hydrolyzed collagen or collagen peptides; and
5) 0.01-5% Hydroxyethylcellulose, preferably 0.1-0.5, or 0.1%.

It will be understood that any component defined herein as being included may be explicitly excluded from the claimed invention by way of proviso or negative limitation. In addition, all ranges given herein include the end of the ranges and also any intermediate range points, whether explicitly stated or not.

Finally, terms of degree such as "substantially", "about" and "approximately" as used herein mean a reasonable amount of deviation of the modified term such that the end result is not significantly changed. These terms of degree should be construed as including a deviation of at least ±5% of the modified term if this deviation would not negate the meaning of the word it modifies.

### Examples - these examples are non-limiting and encompass embodiments of the invention.

### Example 1

Isotonic solutions of sodium chloride 9g/L in water were prepared containing no preservatives (normal saline) containing the constituents of the present invention. Preparation: to 1 liter of distilled water was added 9 grams of sodium chloride. The solution is 0.9% sodium chloride. The pH was 4.5-7.0. Solutions were prepared with the different amounts shown below.

To a 0.9% saline solution was added:
1) 0.1 % PHMB; and
2) 1-10 mM EDTA or 1.0mM acid (EDTA); and
3) 0-1%, 1-3%, 2-4% glycerin, or 2.5% glycerin;
4) 0.001-0.1% hydrolyzed collagen or denatured collagen, in amounts of 0.05% or 0.01-0.1% and collagen peptides in amount of 0.05% was added. When using denatured collagen, gelatin is preferably added as 0.02%.
5) Chondroitin-6-sulfate (0.05% (w/v); and hyaluronic acid (0.1% (w/v) were added to the solution of the invention.
6) 0.01-5% hydroxyethylcellulose (HEC), preferably 0.1-0.5, most preferably 0.1%.

In 5) and 6) the chondroitin-6-sulfate and HEC were optional.

The resulting solution was clear and isotonic and had antibacterial and metal chelating properties as applied on wounds.

### Example 2

Irrigation solutions of this invention comprising phosphate buffered saline (PBS) were prepared. One non-limiting preparation for PBS is described below.

A 10 liter stock of 10x PBS was prepared by dissolving the following:
- 800 g NaCl,
- 20 g KCl,
- 144 g Na₂HPO₄ · 2H₂O
- 24 g KH₂PO₄
- 8 L of distilled water.

After complete mixing, water was added to final solution to 10 L. The pH of the 10X stock was approximately 6.8, but when diluted to 1x PBS to approximately 7.4. If necessary, the pH can be adjusted using hydrochloric acid or sodium hydroxide. Solutions were prepared with the different amounts shown below.

To 1L of the PBS solution was added:
1) 0.1 % PHMB;
2) 0-50 mM EDTA, optimally 1.0mM acid (EDTA);
3) 0-1%, 1-3%, 2-4% glycerin, or 2.5% glycerin;
4) 0.001-0.1% hydrolyzed collagen or denatured collagen, 0.05%, or 0.01-0.1% collagen peptides, or 0.05% was optionally added; when used denatured collagen, gelatin was preferably added as 0.02%;
5) optionally, Chondroitin-6-sulfate (0.05% (w/v); and hyaluronic acid (0.1% (w/v) were added to the solution; and
6) optionally, 0.01-5% Hydroxyethylcellulose, 0.1-0.5, or 0.1%.

The resulting solution was clear and isotonic and had antibacterial properties.

### Example 3

Irrigation solutions were prepared with the different amounts shown below. Into a saline solution was added:
1) 0.01-0.05 % PHMB, preferably 0.1 %-0.5 PHMB and more preferably 0.1% PHMB;
2) 0-50 mM sodium ethylenediamine tetra-acetic acid (EDTA), optimally 10mM (EDTA);
3) 0-1%, 1-4%, 2-3.5 % glycerin, or 2.5% glycerin;
4) 0.1-0.1% hydrolyzed collagen or collagen peptides was added.
5) 0.01-5% Hydroxyethylcellulose, 0.1-0.5, or 0.1% was added.

The resulting solution was clear and isotonic and had antibacterial and metal chelating properties. Longer use aided in facilitating wound/incision healing.

The above formulations are in no way limiting and appropriate combinations and amounts of the constituents may be varied. The irrigation solutions of the present invention are optically clear, non-antigenic, non-toxic and is isotonic such that they do not alter body chemistry or affect the viability of body cells in the wound bed or operative area. The solutions of the present invention are inexpensive and not viscous or sticky, so that they may be used in substantial amounts. The solution can be easily sterilized by appropriate means well-known in the art, and may be stored for extended periods.

## Claims

1. An isotonic irrigation solution comprising:
- saline;
- antimicrobial;
- chelator;
- surfactant; and
- collagenous biological enhancer providing exposed RGD motifs for cellular interaction.

2. The irrigation solution of claim 1, wherein:
said saline comprises physiological saline, saline containing magnesium and/or calcium and phosphate buffered saline (PBS);
said antimicrobial is selected from the group consisting of polyhexamethylene biguanide (PHMB), triclosan, octenidine, PVP-iodine, chlorhexidine digluconate, silver, hypochlorus acid, sodium hypochlorite and any combination thereof, preferably PHMB;
said chelator has preservative qualities and is selected from the group consisting of ethylenediaine tetra-acetic acid (EDTA), disodium EDTA, trisodium EDTA, ethyleneglycotetraacetic acid, Diethylenetriaminepentaacetic acid (DTPA), N,N-bis(carboxymethyl)glycine (NTA), and combinations thereof, preferably EDTA;
said surfactant is selected from the group consisting of betaine, glycerine and combinations thereof, preferably glycerine;
wherein said collagenous biological enhancer is selected from the group consisting of hydrolyzed collagen, collagen peptides. denatured collagen, monomeric collagen and combinations thereof, preferably gelatin.

3. The irrigation solution of claim 1 or 2, wherein said antimicrobial is present in an amount selected from the group consisting of about 0.001%-0.5 % w/v, about 0.1 %-0.5% w/v and about 0.1% w/v, preferably 0.1% w/v PHMB.

4. The irrigation solution of claim 1, 2 or 3, wherein said chelator is present in an amount selected from about 0.01mM up to about 50mM; about 1.0 mM to 10mM; and about 1.0mM, preferably about 1.0mM EDTA.

5. The irrigation solution of any one of claims 1 to 4, wherein said surfactant is present in an amount selected from up to about 4% by weight, about 0.1% to about 4%; about 0.1% to about 3%; about 0.1% to about 2.5%; about 1% to about 3% or 2.5%, preferably 2.5% glycerine.

6. The irrigation solution of any one of claims 1 to 6, wherein said collagenous biological enhancer is present in an amount of about 0.001 to about 1.0%, about 0.05% to about 1.0%, about 0.1% , about 0.05% or about 0.02%.

7. The irrigation solution of any one of claims 1 to 5, wherein said collagenous biological enhancer facilitates wound/incision healing.

8. The irrigation solution of any one of claims 1 to 7, further comprising about 0.01% to about 4% glycosaminoglycans selected from the group consisting of chondroitin-6-sulphate, hyaluronic acid and mixtures thereof.

9. The irrigation solution of claim 8, wherein said glycosaminoglycans provided is 0.05% chondroitin-6-sulphate and 0.01% hyaluronic acid.

10. The irrigation solution of any one of claims 1 to 9, further comprising a thickening agent, preferably hydroxyethylcellulose (HEC).

11. The irrigation solution of claim 10, wherein said thickening agent is provided in an amount of about 0.01% to about 5%, about 0.1% to about 0.5%, or about 1.0% w/v.

12. The irrigation solution of any one of claims 1 to 11 having a pH of about 5.0 to about 7.5, about 6.5 to about 7.5 or about 6.8 to about 7.4, further wherein said solution is one or more of sterilized, anti-microbial, biologically enhancing, optically clear, essentially non-electrolytic, essentially non-antigenic, non-toxic, and essentially non-hemolytic.

13. The irrigation solution of any one of claims 1 to 12, provided in a ready to use format, or in a concentrated format and/or provided soaked in a suitable material selected from gauze and sponge and optionally packaged for use.

14. The irrigation solution of any one of claims 1 to 13, provided as a kit with instructions for use.

15. The irrigation solution of any one of claims 1 to 14 for use as a medicament, preferably for use in treating a wound or surgical incision, or as a lavage, more preferably to maintain the viability of cells/tissues of a wound, to remove non-viable or necrotic tissue of a wound, to remove contamination materials or microbes form a wound, to enhance the removal of biofilms of a wound, to enhance visualization of a wound, to provide tissue nutrients and enzyme inhibitors to a wound, to chelate metal ions of a wound or to establish a wound bed and activate host cells to support healing of said wound.
